# EUROPEAN PATENT SPECIFICATION

(11) **EP 3 781 542 B1**
(45) Date of publication and mention of the grant of the patent: **18.05.2022**
(21) Application number: 19716916.2
(22) Date of filing: 15.04.2019
(51) Int. Cl.: C07C 46/06, C07C 50/02, C07C 50/12, C07C 50/04

(54) **OXIDATION OF ALKYLATED P-HYDROQUINONES IN AQUEOUS SOLUTIONS BY HYDROGEN PEROXIDE**
OXIDATION VON ALKYLIERTEN P-HYDROCHINONEN IN WÄSSRIGEN LÖSUNGEN DURCH WASSERSTOFFPEROXID
OXYDATION DE P-HYDROQUINONES ALKYLÉS DANS DES SOLUTIONS AQUEUSES PAR DU PEROXYDE D'HYDROGÈNE

(30) Priority: 17.04.2018 EP 18167718
(43) Date of publication of application: 24.02.2021
(73) Proprietor: DSM IP Assets B.V., 6411 TE Heerlen (NL)
(72) Inventor: BONRATH, Werner, 4303 Kaiseraugst (CH); MUELLER, Marc-André, 4303 Kaiseraugst (CH); NETSCHER, Thomas, 4303 Kaiseraugst (CH); SMUDA, Christina, 4303 Kaiseraugst (CH); ZWEIFEL, Theodor, 4303 Kaiseraugst (CH)
(74) Representative: Dux, Roland
(86) International application number: PCT/EP2019/059589
(87) International publication number: WO 2019/201820

(56) References cited:
- US-A- 5 712 416
- SHIMIZU M ET AL: "A convenient synthesis of alkyl-substituted p-benzoquinones from phenols by a H2O2/heteropolyacid system", TETRAHEDRON LETTERS, ELSEVIER, AMSTERDAM, NL, vol. 30, no. 4, 1 January 1989 (1989-01-01), pages 471-474, XP002384633, ISSN: 0040-4039, DOI: 10.1016/S0040-4039(00)95231-6

## Description

The present invention relates to the oxidation of alkylated p-hydroquinones.

### Background of the invention

Alkylated p-hydroquinones and alkylated p-benzoquinones are important intermediates in a series of industrial applications. Particularly, they are used in the synthesis of tocopherols.

The oxidation of alkylated p-hydroquinones to obtain the respective alkylated p-benzoquinones is particularly interesting for the industry.

Oxidation of phenols per se is known to the person skilled in the art. For example, EP 0 659 727 A1 discloses a process for the preparation of benzoquinones by oxidation of phenols in the presence of a complex of iron, manganese or chromium with tetraaza[14]annulene as polydentate complexing ligand.

M. Shimizu, in Tetrahedron Lett., 1989, 30, 471-474, discusses the oxidation of a phenol carried out by H₂O₂ in the presence of a heteropolyacid.

WO 2015/169898 A1 discloses the oxidation of 2,6-dimethylphenol to 2,6-dimethylbenzoquinone by means of O₂ in the presence of a copper salt.

WO 2015/000767 A1 discloses the oxidation of 2,3,6-trimethylphenol to 2,3,6-trimethylbenzoquinone by means of O₂ in the presence of a copper salt and a secondary aliphatic acyclic alcohol with 6 or more carbon atoms.

These documents, however, all relate to the oxidation of phenols, i.e. compounds carrying only one hydroxyl group at the aromatic ring, and not to the oxidation of hydroquinones, i.e. carrying two hydroxyl groups in para positions at the aromatic ring.

R.G. Jones et al. in J. Am. Chem. Soc. 1945, 67, 1034-1035 disclose the preparation of 2,5-dihydroxyquinone by oxidation of hydroquinone in highly concentrated (50 %) sodium hydroxide solution with 30 % hydrogen peroxide. These harsh reaction conditions, however, lead to significant safety risks.

M. Shimizu et al., in Tetrahedron Letters 1989, 30, 471-474 disclose the catalytic oxidation of trimethylhydroquinone to trimethyl-p-benzoquinones by H₂O₂ in the presence of heteropolyacids. Heteropolyacids are very strong acids and stronger acids than H₂SO₄ or HNO₃ or even HClO₄. The low pH, however, leads to difficulties in handling heteropolyacids and leads to significant problems of corrosion, particularly if the reaction would be performed in an industrial scale. Finally, major quantities of base need to be employed when neutralizing the residues after termination of the reaction. This is economically and environmentally very disadvantageous

EP 0 659 727 A1 discloses the oxidation of phenols by hydrogen peroxide or oxygen in the presence of an oxygen-transferring catalyst selected from the group consisting of iron, manganese and chromium tetraaza[14]annulenes to the respective benzoquinones in a of large amount of diluents, particularly acetic acid.

Organic diluents, and particularly acetic acid, are inflammable organic liquids and are as a result thereof critical in fire hazards or explosions. The use of large amounts of acetic acid in presence of oxygen is not only disadvantageous in view of fire hazard but also in view of corrosion hazard.

Oxygen is a gas. The use of a gas in chemical reaction offers significant technical challenges. It is therefore very convenient to use a suitable liquid oxidant for an oxidation reaction.

Metal complexes of tetraaza[14]annulenes are very expensive, not readily commercially available. Their eco-impact is also to be assessed rather as critical. Finally, it has been shown, that metal complexes are only of limited interest for oxidation reactions as the metal complexes, particularly the ligands involved, are prone to be oxidized themselves. This of course is very disadvantageous in view of efficiency and re-cyclability of these metal compounds.

Finally, the solubility of the metal complexes in water is a very important limiting factor for the oxidation to occur in a cost-efficient way.

All these factors show, that there is a great need in industry for a save, economically friendly and cost-efficient method of oxidation of alkylated p-hydroquinones to the corresponding alkylated p-benzoquinones.

### Summary of the invention

The present invention offers an improved process of oxidation of alkylated p-hydroquinones to the corresponding alkylated p-benzoquinones.

It has been shown that this process leads to a high yield and excellent selectivity and reaction rate. The invention enables a very safe production particularly in regard to fire hazards. By using aqueous hydrogen peroxide as oxidizing agent the invention can offer an ecologically friendly and particular inexpensive and safe oxidation process.

Particularly, it has been shown that the manufacturing process is also suitable for aqueous systems at very low concentrations of the alkylated p-hydroquinones.

Further aspects of the invention are subject of further independent claims. Particularly preferred embodiments are subject of dependent claims.

### Detailed description of the invention

In a first aspect, the present invention relates to a process of manufacturing a compound of formula (I) 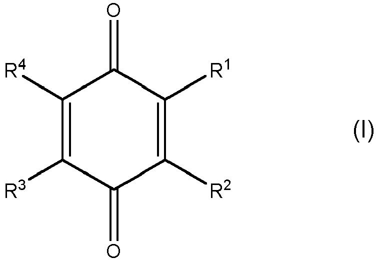 by oxidation of a compound of formula (II) 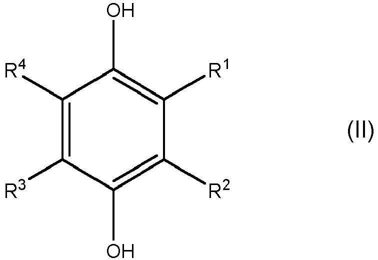 using aqueous H₂O₂ as oxidizing agent in the presence of a transition metal salt, which does not comprise any polydentate ligand bound to the metal ion, and an organic acid and water;
wherein R¹, R², R³ and R⁴ independently represent either H or a C₁₋₄-alkyl group or R¹ and R² form together a divalent carbon chain to yield a 6-membered saturated or aromatic ring;
with the proviso that at least one of the residues R¹, R², R³ and R⁴ is different from H;
characterized in that the transition metal is selected from the group consisting of Ce, Mn, Fe, Cu and Zn, particularly is selected from the group consisting of Mn, Fe, Cu and Zn.

For sake of clarity, some terms as been used in the present document are defined as follows:
In the present document, a "C_{x-y}-alkyl" group is an alkyl group comprising x to y carbon atoms, i.e., for example, a C₁₋₃-alkyl group is an alkyl group comprising 1 to 3 carbon atoms. The alkyl group can be linear or branched. For example -CH(CH₃)-CH₂-CH₃ is considered as a C₄-alkyl group.

In case identical labels for symbols or groups are present in several formulae, in the present document, the definition of said group or symbol made in the context of one specific formula applies also to other formulae which comprises the same said label.

The term "inert" in "inert organic solvent" means that under the conditions of the reaction the solvent undergoes no chemical reaction or lead to any change of pH.

The term "essentially no inert organic solvent" as used in the present documents means that ideally no inert organic solvents are present at all.

However, "essentially no inert organic solvent" means also that only minor traces in an amount not exceeding 3 % by weight, preferably not exceeding 1 % by weight, can be present. Large amounts of inert solvents, however, would negatively impact the present invention.

The groups R¹, R², R³ and R⁴ independently represent either H or a C₁₋₄-alkyl group or R¹ and R² form together a divalent carbon chain to yield a 6-membered saturated or aromatic ring with the proviso that at least one of the residues R¹, R², R³ and R⁴ is different from H.

In one embodiment, R¹, R², R³ and R⁴ independently represent either H or a C₁₋₄-alkyl group with the proviso that at least one of the residues R¹, R², R³ and R⁴ is different from H.

Particularly, R¹, R², R³ and R⁴ independently represent either H or CH₃.

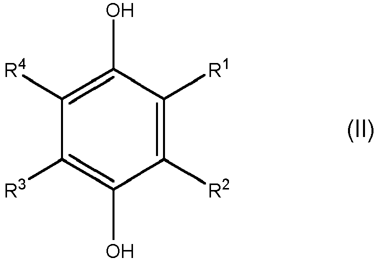

Preferred are the following combinations of R¹, R², R³ and R⁴:
- R⁴ = H and R¹ = R² = R³ = C₁₋₄-alkyl group; particularly CH₃
- R⁴ = R² = H and R¹ = R³ = C₁₋₄-alkyl group; particularly CH₃
- R⁴ = R³ = H and R¹ = R² = C₁₋₄-alkyl group; particularly CH₃
- R⁴ = R³ = R² = H and R¹ = C₁₋₄-alkyl group; particularly CH₃
- R³ = R² = H and R¹ = R⁴ = C₁₋₄-alkyl group; particularly CH₃.

Preferred is that R⁴=H and R¹ = R² = R³ = C₁₋₄-alkyl group; particularly R¹ = R² = R³ = CH₃.

Most preferred is, therefore, the combination of R⁴ = H and R¹ = R² = R³ = CH₃.

In other words, the most preferred compound of formula (I) is 2,3,5-trimethylbenzoquinone (=2,3,5-trimethylcyclohexa-2,5-diene-1,4-dione) and the most preferred compound of formula (II) is 2,3,5-trimethylhydroquinone (=2,3,5-trimethylbenzene-1,4-diol).

The compound of formula (II) is known to the person skilled in the art and easily accessible.

In another embodiment, R¹ and R² form together a divalent carbon chain to yield a 6-membered saturated or aromatic ring and R³ and R⁴ independently represent either H or a C₁₋₄-alkyl group.

Preferably, R¹ and R² form together a divalent carbon chain to yield a 6-membered aromatic ring.

In this embodiment, preferably the compound of formula (II) is a compound of formula (II-A) or (II-B)

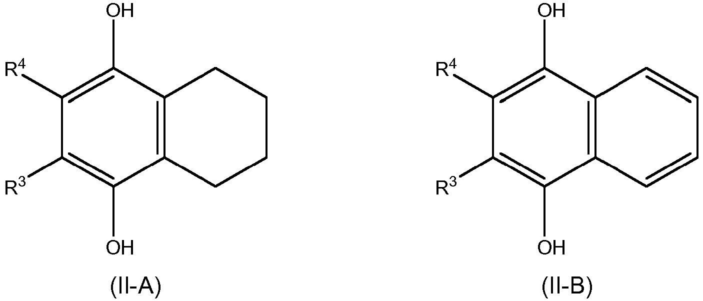

Accordingly, the preferred compound of formula (I) for this embodiment is a compound of formula (I-A) or (I-B).

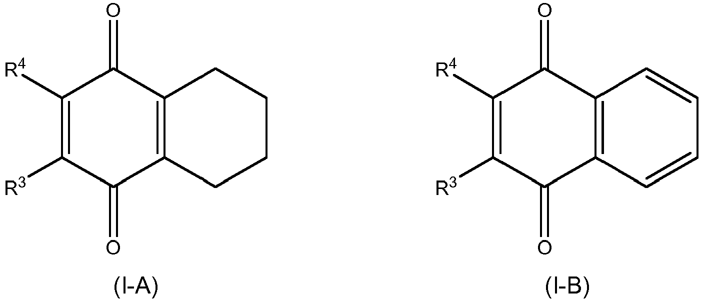

It is preferred that for this embodiment, compound of formula (I) is a compound of (I-B) and compound of formula (II) is a compound of formula (II-B).

Preferably, compound of formula (I-B) is 2-methyl-1,4-naphthoquinone (also called menadione [58-27-5], vitamin K3) which is an important intermediate for the synthesis of vitamins K1 and K2.

The compound of formula (II) is present in an amount of typically 0.3 to 10 % by weight, particularly 0.8 to 5.0 % by weight, preferably 0.8 - 2.5 % by weight, based on the weight of the complete reaction mixture at the beginning of the oxidation.

The above process is carried out in the presence of a transition metal salt which does not comprise any polydentate ligand bound to the metal ion. Said transition metal salt is selected from the group consisting of Ce, Mn, Fe, Cu and Zn, particularly is selected from the group consisting of Mn, Fe, Cu and Zn.

Preferably, the transition metal salt is a salt of Mn(II), Mn(IV), Fe(II), Fe(III), Cu(I), Cu(II) or Zn(II), particularly of Fe(II) or Fe(III). Most preferred is that the transition metal salt is a salt of Fe(II) or Fe(III).

Ferric salts (Fe(III)) are preferred over ferrous salts (Fe(II)). It has been shown that ferrous salts give slightly lower yields than ferric salts under the same conditions.

The transition metal salt is preferably soluble in water. Particularly, the solubility of the transition metal salt in water is at least so high that it forms a clear solution at the temperature and amounts of water used for the oxidation reaction.

The transition metal salt is preferably a sulphate, chloride, carboxylate, particularly an acetate, of the transition metal, particularly of a sulphate, chloride, carboxylate, particularly an acetate, of Mn(II), Mn(IV), Fe(II), Fe(III), Cu(I), Cu(II) or Zn(II).

The amount of transition metal salt is present at the beginning of the oxidation in an amount of at least 0.02 mol-%, particularly in an amount of 0.02 - 2 mol-%, more particularly in an amount of 0.3 - 1.0 mol-%, based on the amount of the compound of formula (II).

The transition metal salt can be recycled and be reused.

The process is usually carried out at a temperature of from 40°C to 95°C, particularly between 50°C and 85°C, preferably between 55°C and 70°C.

The above process is carried out in the presence of an organic acid. Preferably, the organic acid is selected from the group consisting of acetic acid, adipic acid, lactic acid, oxalic acid, citric acid, particularly acetic acid.

The amount of organic acid added is in an amount that the pH of the reaction mixture is in the range of 4.8 -6.8, particularly 5 - 6.8, preferably 5.5 - 6.2.

It is preferred that the amounts of organic acid added is 0.15 - 3.5 mol-equivalent, preferably 1.5 -2.5 mol-equivalent, based on the amount of formula (II).

It is important to stress that in the present invention only small amounts of any organic acids are used. The reaction mixtures of the present inventions are advantageous in view of handling, particularly in view of flammability, which is present in the state of the art technology when reaction mixtures are handled in the presence of large amounts of highly volatile organic substances, such as solvents or organic acids, in the presence of an oxidant.

Furthermore, performing the reaction at a very low pH, particularly at a pH of lower than 3.5, or even lower than 3, is very disadvantageous due to the risk of corrosion of the equipment used or health risk for the employees involved in said production process. Finally, when the reaction would be made under strongly acid condition, major quantities of base would be needed when neutralizing the residues after termination of the reaction, which would be, therefore also very disadvantageous from an economic and/or environmental point of view. With the present invention, it has been observed that the reaction excellently proceeds also at conditions at slightly acid pH or even at neutral pH.

Therefore, it is preferred that the oxidation takes place at a pH of 5 - 6.8, particularly of 5.5 - 6.2.

The oxidation of the compound of formula (II) is carried out by means of aqueous H₂O₂ as oxidizing agent. Preferably, a 10 - 80 % by weight, preferably 20 - 50 % by weight, aqueous solution of H₂O₂ is used.

Particularly, it has been found that oxidation of the compound of formula (II) is efficient at even very high amounts of water. It has been found that the present invention is particularly suitable for oxidation of compound of formula (II) in cases where the amount of water is more than 90 % by weight, preferably more than 95 %, of the reaction mixture.

Hence, it has been found that the present invention offers a very valuable method for the oxidation of compound of formula (II) also in very diluted forms of compound of formula (II) in aqueous systems as they occur for example in work-up of aqueous washing or extractions during productions based on compound of formula (I) or (II) or derivatives thereof.

It is preferred that essentially no inert organic solvent is present during the oxidation. The presence of inert organic solvents, particularly in significant amounts would be negatively impacting the present invention. Inert organic solvents are negatively influencing as they would lead to problems in the solubility of the transition metal salts. Furthermore, their high volatility and flammability would significantly increase the fire hazard.

Particularly, the oxidation is carried out in such a manner that the sum of the weight of
a) the compound of formula (II)
b) H₂O₂
c) the transition metal salt, which does not comprise any polydentate ligand bound to the metal ion
d) the organic acid
e) water
is more than 95 %, preferably more than 98%, more preferably more than 99 %, of the weight of the complete reaction mixture at the beginning of the oxidation.

In a preferred way of oxidation, a mixture of at least the compound of formula (II), water and the transition metal salt and the organic acid is prepared and aqueous H₂O₂ is added to said mixture. It is preferred that the aqueous hydrogen peroxide is added dropwise or at least in small portions under stirring at elevated temperatures, i.e. at temperatures above 25°C, particularly at a temperature of between 50 and 70°C. The addition rate can vary and depends amongst other parameters particularly on the size of the reaction vessel and stirring rate.

It is preferred that the oxidation is carried out in the absence of any complexing agent or any complex having bound a complexing agent as ligand.

The above process is preferably carried out in a continuous way, i.e. that the process is preferably a continuous process. In this embodiment, the process is carried out in a suitable reaction vessel, known per se for continuous reactions.

It has been shown that the process described above leads to a high yield and excellent selectivity and reaction rate. It enables offering a very safe production process.

The process using hydrogen peroxide as oxidizing agent has the advantage that the present oxidation process is ecologically friendly as the only (by-)product formed in the oxidation reaction from hydrogen peroxide is water.

Particularly, it has been shown that the process is also suitable for aqueous systems in very low concentrations of the alkylated p-hydroquinones.

### Examples

The present invention is further illustrated by the following experiments.

### General procedure for the oxidation of 2,3,5-trimethylhydroquinone (TMHQ)

A sulfonation flask equipped with a reflux condenser and an overhead stirrer was charged with H₂O (99.0 mL) and argon was bubbled through the solvent for 10 minutes. The equipment was evacuated and back-filled with argon three-times and TMHQ (1.00 g, 6.45 mmol, 1.00 eq) was added. An aqueous NaOH solution (10 %, 5.00 mL) was added to the suspension to adjust the pH value to -12 and the reaction mixture was stirred for 15 minutes until all TMHQ had dissolved. The corresponding amount of iron-salt was added and the reaction mixture was warmed to 60°C. Acetic acid (0.2 equivalents) and HCI (25 %, 1.4 mL) was added to adjust the pH to ~6. Aqueous H₂O₂ solution (646 µL [30.6 % by weight], 6.45 mmol, 1.00 eq) was added over a period of 30 min and the reaction mixture was stirred for further 15 min at 60 °C. The reaction mixture was cooled to ambient temperature and extracted with toluene (2×50 mL). The combined organic layers were washed with H₂O (1×30 mL) and concentrated at 50°C/25 mbar to obtain a yellow liquid being identified to be 2,3,5-trimethylbenzoquinone (TMQ). The obtained yields for TMQ have been compiled in table 1.

**Table 1. Oxidation of TMHQ to TMQ by aqueous H₂O₂.**

| **Example** | **Acid** | **Fe salt** | **Ox ^{c)}** | **Fe [mol-%]** | **Yield TMQ [%]** |
|---|---|---|---|---|---|
| ***1*** | Acetic acid | Fe₂(SO₄)₃×6H₂O | Fe(III) | 0.2 | 77 |
| ***2*** | Acetic acid | Fe₂(SO₄)₃×6H₂O | Fe(III) | 0.4 | 82 |
| ***3*** | Acetic acid | Fe₂(SO₄)₃×6H₂O | Fe(III) | 0.8 | 84 |
| ***4*** | Acetic acid | Fe(SO₄)×7H₂O | Fe(II) | 0.4 | 73 |
| ***5*** | Acetic acid | FeCl₃×6H₂O | Fe(III) | 0.4 | 81 |
| ***6*** | Adipic acid | Fe₂(SO₄)₃×6H₂O | Fe(III) | 0.4 | 82 |
| ***Ref.1*** ^{a)} | Acetic acid | | | | 11.7 |
| ***Ref. 2***^{b)} | | | | | < 5 |

| | | | | | |
|---|---|---|---|---|---|
| ^{a)}oxidation by air (1 bar), reaction time 2¼ hour. ^{b)}oxidation by NaOCI (1.1 equivalents) at pH = 8. ^{c)}Ox = oxidation state of iron salt used. | | | | | |

## Claims

1. A process of manufacturing a compound of formula (I) 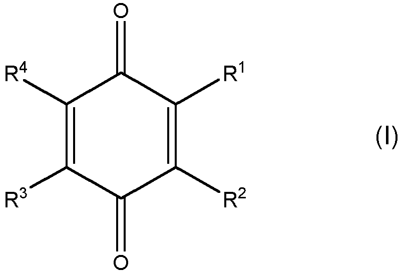 by oxidation of a compound of formula (II) 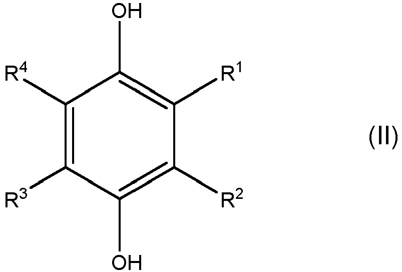 using aqueous H₂O₂ as oxidizing agent
in the presence of a transition metal salt, which does not comprise any polydentate ligand bound to the metal ion, and an organic acid and water; wherein R¹, R², R³ and R⁴ independently represent either H or a C₁₋₄-alkyl group or R¹ and R² form together a divalent carbon chain to yield a 6-membered saturated or aromatic ring;
with the proviso that at least one of the residues R¹, R², R³ and R⁴ is different from H,
**characterized in that** the transition metal is selected from the group consisting of Ce, Mn, Fe, Cu and Zn, particularly is selected from the group consisting of Mn, Fe, Cu and Zn.

2. The process according to claim 1, **characterized in that** an inert organic solvent is not present or present in an amount not exceeding 3 % by weight, preferably not exceeding 1 % by weight, during the oxidation;
and that that under the conditions of the reaction, the inert solvent does not undergo any chemical reaction or lead to any change of pH.

3. The process according to claim 1 or 2, **characterized in that** R⁴=H and R¹ = R² = R³ = C₁₋₄-alkyl group; particularly R¹ = R² = R³ = CH₃.

4. The process according to any one of the preceding claims, **characterized in that** the transition metal salt is a salt of Mn(II), Mn(IV), Fe(II), Fe(III), Cu(I), Cu(II) or Zn(II), particularly of Fe(II) or Fe(III).

5. The process according to any one of the preceding claims, **characterized in that** the organic acid is selected from the group consisting of acetic acid, adipic acid, lactic acid, oxalic acid, citric acid, particularly acetic acid.

6. The process according to any one of the preceding claims, **characterized in that** the amount of water is more than 90 % by weight, preferably more than 95 % of the reaction mixture.

7. The process according to any one of the preceding claims, **characterized in that** the sum of the weight of
a) the compound of formula (II)
b) H₂O₂
c) the transition metal salt, which does not comprise any polydentate ligand bound to the metal ion
d) the organic acid
e) water
is more than 95 %, preferably more than 98 %, more preferably more than 99 %, of the weight of the complete reaction mixture at the beginning of the oxidation.

8. The process according to any one of the preceding claims, **characterized in that** the compound of formula (II) is present in an amount of 0.3 to 10 % by weight, particularly 0.8 to 5 % by weight, preferably 0.8 - 2 % by weight, based on the weight of the complete reaction mixture at the beginning of the oxidation.

9. The process according to any one of the preceding claims, **characterized in that** the transition metal of the transition metal salt is present at the beginning of the oxidation in an amount of at least 0.02 mol-%, particularly in an amount of 0.02 - 2 mol-%, more particularly in an amount of 0.3 - 1.0 mol-%, based on the amount of the compound of formula (II).

10. The process according to any one of the preceding claims, **characterized in that** the oxidation takes place at a pH of 5 - 6.8, particularly of 5.5 - 6.2.

11. The process according to any one of the preceding claims, **characterized in that** the process is a continuous process.

## Patentansprüche

1. Verfahren zur Herstellung einer Verbindung der Formel (I) 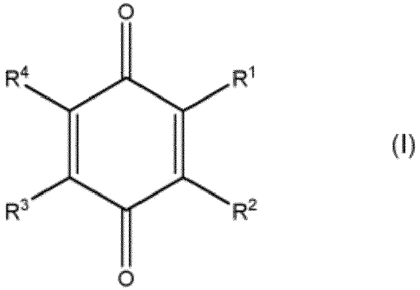 durch Oxidation einer Verbindung der Formel (II) 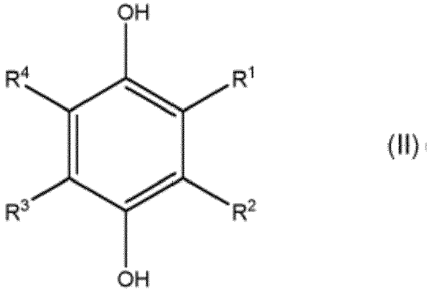 unter Verwendung von wässrigem H₂O₂ als Oxidationsmittel
in Gegenwart von einem Übergangsmetallsalz, das keinen an das Metallion gebundenen mehrzähnigen Liganden umfasst, und einer organischen Säure und Wasser;
wobei R¹, R², R³ und R⁴ unabhängig voneinander für H oder eine C₁₋₄-Alkylgruppe stehen oder R¹ und R² zusammen eine zweiwertige Kohlenwasserstoffkette unter Erhalt eines 6-gliedrigen ungesättigten oder aromatischen Rings bilden;
mit der Maßgabe, dass mindestens einer der Reste R¹, R², R³ und R⁴ von H verschieden ist,
**dadurch gekennzeichnet, dass** das Übergangsmetall aus der Gruppe bestehend aus Ce, Mn, Fe, Cu und Zn ausgewählt ist und insbesondere aus der Gruppe bestehend aus Mn, Fe, Cu und Zn ausgewählt ist.

2. Verfahren nach Anspruch 1, **dadurch gekennzeichnet, dass** während der Oxidation kein inertes organisches Lösungsmittel vorliegt oder ein inertes organisches Lösungsmittel in einer Menge von nicht mehr als 3 Gew.-%, vorzugsweise nicht mehr als 1 Gew.-%, vorliegt
und dass das inerte Lösungsmittel unter den Bedingungen der Reaktion keine chemische Reaktion eingeht oder zu einer Änderung des pH-Werts führt.

3. Verfahren nach Anspruch 1 oder 2, **dadurch gekennzeichnet, dass** R⁴ = H und R¹ = R² = R³ = C₁₋₄-Alkylgruppe; insbesondere R¹ = R² = R³ = CH₃.

4. Verfahren nach einem der vorhergehenden Ansprüche, **dadurch gekennzeichnet, dass** es sich bei dem Übergangsmetallsalz um ein Salz von Mn(II), Mn(IV), Fe(II), Fe(III), Cu(I), Cu(II) oder Zn(II), insbesondere von Fe(II) oder Fe(III), handelt.

5. Verfahren nach einem der vorhergehenden Ansprüche, **dadurch gekennzeichnet, dass** die organische Säure aus der Gruppe bestehend aus Essigsäure, Adipinsäure, Milchsäure, Oxalsäure, Citronensäure, insbesondere Essigsäure, ausgewählt wird.

6. Verfahren nach einem der vorhergehenden Ansprüche, **dadurch gekennzeichnet, dass** die Wassermenge mehr als 90 Gew.-%, vorzugsweise mehr als 95 Gew.-%, der Reaktionsmischung ausmacht.

7. Verfahren nach einem der vorhergehenden Ansprüche, **dadurch gekennzeichnet, dass** die Summe des Gewichts von
a) der Verbindung der Formel (II),
b) H₂O₂,
c) dem Übergangsmetallsalz, das keinen an das Metallion gebundenen mehrzähnigen Liganden umfasst,
d) der organischen Säure,
e) Wasser
mehr als 95 %, vorzugsweise mehr als 98 %, weiter bevorzugt mehr als 99 %, des Gewichts der kompletten Reaktionsmischung zu Beginn der Oxidation beträgt.

8. Verfahren nach einem der vorhergehenden Ansprüche, **dadurch gekennzeichnet, dass** die Verbindung der Formel (II) in einer Menge von 0,3 bis 10 Gew.-%, insbesondere 0,8 bis 5 Gew.-%, vorzugsweise 0,8-2 Gew.-%, bezogen auf das Gewicht der kompletten Reaktionsmischung zu Beginn der Oxidation, vorliegt.

9. Verfahren nach einem der vorhergehenden Ansprüche, **dadurch gekennzeichnet, dass** das Übergangsmetall des Übergangsmetallsalzes zu Beginn der Oxidation in einer Menge von mindestens 0,02 Mol-%, insbesondere in einer Menge von 0,02-2 Mol-%, spezieller in einer Menge von 0,3-1,0 Mol-%, bezogen auf die Menge der Verbindung der Formel (II), vorliegt.

10. Verfahren nach einem der vorhergehenden Ansprüche, **dadurch gekennzeichnet, dass** die Oxidation bei einem pH-Wert von 5-6,8, insbesondere von 5,5-6,2, stattfindet.

11. Verfahren nach einem der vorhergehenden Ansprüche, **dadurch gekennzeichnet, dass** es sich bei dem Verfahren um ein kontinuierliches Verfahren handelt.

## Revendications

1. Procédé de fabrication d'un composé de formule (I) 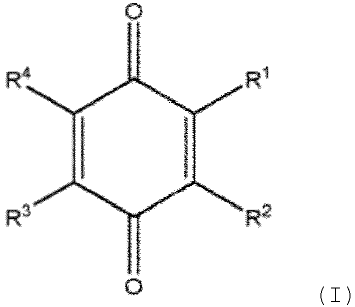 par oxydation d'un composé de formule (II) 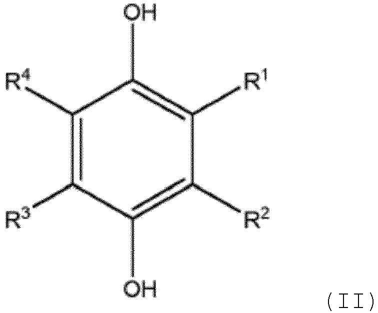 en utilisant du H₂O₂ aqueux en tant qu'agent oxydant en la présence d'un sel de métal de transition, qui ne comprend aucun ligand polydentate lié à l'ion métallique, et un acide organique et de l'eau ;
R¹, R², R³ et R⁴ représentant indépendamment soit H, soit un groupe C₁₋₄-alkyle ou R¹ et R² formant ensemble une chaîne carbonée divalente pour donner un cycle saturé ou aromatique à 6 chaînons ;
à la condition qu'au moins l'un des radicaux R¹, R², R³ et R⁴ soit différent de H,
**caractérisé en ce que** le métal de transition est choisi dans le groupe constitué par Ce, Mn, Fe, Cu et Zn, particulièrement est choisi dans le groupe constitué par Mn, Fe, Cu et Zn.

2. Procédé selon la revendication 1, **caractérisé en ce qu'**un solvant organique inerte n'est pas présent ou est présent en une quantité ne dépassant pas 3 % en poids, préférablement ne dépassant pas 1 % en poids, pendant l'oxydation ;
et **en ce que** dans les conditions de la réaction, le solvant inerte ne subit aucune réaction chimique ou ne conduit à aucun changement de pH.

3. Procédé selon la revendication 1 ou 2, **caractérisé en ce que** R⁴ = H et R¹ = R² = R³ = groupe C₁₋₄-alkyle ; particulièrement R¹ = R² = R³ = CH₃.

4. Procédé selon l'une quelconque des revendications précédentes, **caractérisé en ce que** le sel de métal de transition est un sel de Mn(II), Mn(IV), Fe(II), Fe(III), Cu(I), Cu(II) ou Zn(II), particulièrement de Fe(II) ou Fe (III) .

5. Procédé selon l'une quelconque des revendications précédentes, **caractérisé en ce que** l'acide organique est choisi dans le groupe constitué par l'acide acétique, l'acide adipique, l'acide lactique, l'acide oxalique, l'acide citrique, particulièrement l'acide acétique.

6. Procédé selon l'une quelconque des revendications précédentes, **caractérisé en ce que** la quantité d'eau est supérieure à 90 % en poids, préférablement supérieure à 95 % du mélange réactionnel.

7. Procédé selon l'une quelconque des revendications précédentes, **caractérisé en ce que** la somme du poids
a) du composé de formule (II)
b) de H₂O₂
c) du sel de métal de transition, qui ne comprend aucun ligand polydentate lié à l'ion métallique
d) de l'acide organique
e) de l'eau
est supérieure à 95 %, préférablement supérieure à 98 %, plus préférablement supérieure à 99 %, du poids du mélange réactionnel total au début de l'oxydation.

8. Procédé selon l'une quelconque des revendications précédentes, **caractérisé en ce que** le composé de formule (II) est présent en une quantité de 0,3 à 10 % en poids, particulièrement de 0,8 à 5 % en poids, préférablement de 0,8 à 2 % en poids, sur la base du poids du mélange réactionnel total au début de l'oxydation.

9. Procédé selon l'une quelconque des revendications précédentes, **caractérisé en ce que** métal de transition du sel de métal de transition est présent au début de l'oxydation en une quantité d'au moins 0,02 % en moles, particulièrement en une quantité de 0,02 à 2 % en moles, plus particulièrement en une quantité de 0,3 à 1,0 % en moles, sur la base de la quantité du composé de formule (II).

10. Procédé selon l'une quelconque des revendications précédentes, **caractérisé en ce que** l'oxydation a lieu à un pH de 5 à 6,8, particulièrement de 5,5 à 6,2.

11. Procédé selon l'une quelconque des revendications précédentes, **caractérisé en ce que** le procédé est un procédé continu.
